# EUROPEAN PATENT SPECIFICATION

(11) **EP 3 351 291 B1**
(45) Date of publication and mention of the grant of the patent: **23.11.2022**
(21) Application number: 17382028.3
(22) Date of filing: 20.01.2017
(51) Int. Cl.: A61N 1/378, H01L 41/113, C12N 5/00, C01G 9/02, H02N 2/18, H01L 41/45

(54) **SELF-GENERATING VOLTAGE DEVICE FOR ELECTRICAL CELL STIMULATION, AND METHOD THEREOF**
VORRICHTUNG FÜR SELBSTERZEUGENDE SPANNUNG ZUR ELEKTRISCHEN ZELLSTIMULIERUNG UND VERFAHREN DAFÜR
DISPOSITIF AUTOGÉNÉRATEUR DE TENSION POUR LA STIMULATION DES CELLULES ÉLECTRIQUES ET PROCÉDÉ ASSOCIÉ

(43) Date of publication of application: 25.07.2018
(73) Proprietor: Consejo Superior de Investigaciones Científicas (CSIC), 28006 Madrid (ES)
(72) Inventor: RODRIGUEZ MURILLO, Gonzalo, 08193 Cerdanyola del Vallès (Barcelona) (ES); ESTEVE TINTÒ, Jaume, 08193 Cerdanyola del Vallès (Barcelona) (ES); VARGAS ESTEVEZ, Carolina, 08193 Cerdanyola del Vallès (Barcelona) (ES)
(74) Representative: Pons Ariño, Angel

(56) References cited:
- WO-A1-2016/207458
- WO-A2-2016/195247
- JP-A- 2006 199 552
- US-A1- 2013 026 969
- US-A1- 2015 182 741
- WANG ET AL: "ZnO nanowire and nanobelt platform for nanotechnology", MATERIALS SCIENCE AND ENGINEERING: R: REPORTS, ELSEVIER, AMSTERDAM, NL, vol. 64, no. 3-4, 3 April 2009 (2009-04-03), pages 33-71, XP026027229, ISSN: 0927-796X, DOI: 10.1016/J.MSER.2009.02.001 [retrieved on 2009-03-09]

## Description

### OBJECT OF THE INVENTION

The invention hereby disclosed belongs to the field of biology and devices intended for biological applications.

The object of the invention provides a new solution to electrically stimulate living biological cells without the needs of electrodes or bulky devices. It takes advantage of the inherent cell forces to create a piezoelectric potential in response able to change the electrical activity of the cell.

### BACKGROUND OF THE INVENTION

Cellular growth and cell stimulation has been studied for decades, different approaches and techniques have been developed in order to provide an efficient methodology for growing and differentiation of cells.

The acceleration and promotion of cell growth is crucial for some technical fields and applications, like biomedicine, in this sense Seunghan Oh, Chiara Daraio, Li-Han Chen, Thomas R. Pisanic, Rita R. Fiñones and Sungho Jin Significantly accelerated osteoblast cell growth on aligned TiO2 nanotubes Journal of Biomedical Materials Research Part A Volume 78A, Issue 1, pages 97-103, July 2006 disclosed Carbon NanoTubes CNTs for supporting cellular growth; this mean using nanostructures for supporting cellular growth, in this scientific paper *Seunghan Oh* et al. disclosed the use of porous CNTs which is advantageous for promoting cellular growth since pores promote attachment of cells.

Electroactive materials are being using to enhance cell cultures in the last years. On the other hand, piezoelectric materials are catching a huge interest because of their utility as key materials for autonomous and energy harvesting systems. A piezoelectric material has the peculiarity of creating an inherent electric field when it is strained (direct piezoelectric effect).

Respective devices are disclosed by US 2015/182741, US 2013/026969 and JP 2006 199552.

In particular, nanostructured materials have surprising properties different than bulk materials due to scaling-down effects. ZnO has become very popular in material science over last few years because of its wide variety of nanostructures and its dual property of being both a semiconducting and piezoelectric material.

Although the term "nanogenerator" (NG) was invented some years ago; in Z. L. Wang, J. H. Song, Piezoelectric nanogenerators based on zinc oxide nanowire arrays., Science 312, 242-6 (2006*)* and Z. L. Wang, ZnO nanowire and nanobelt platform for nanotechnology, Mater. Sci. Eng. RReports 64, 33-71 (2009*)* Prof. ZL Wang demonstrated that a single ZnO nanowire (NW) can generate a certain piezoelectric potential along itself when it is strained. The generated energy output by one nanowire in one discharge event is about 0.05 fJ and the output voltage on the load is around 6-9 mV, for a 5 nN force applied by an AFM tip.

In addition, in the last years, the use of inorganic nanowires to perform rapid analysis of cellular functions have been widely reported, i.e. said use of said nanowire has been disclosed in *M. Kwak, L. Han, J. J. Chen, R. Fan, Interfacing inorganic nanowire arrays and living cells for cellular function analysis, Small, 5600-5610 (2015) .*

Furthermore, it has been demonstrated that dynamic strain induced in piezoelectric nanoparticles can directly generate a depolarization or hyperpolarization of cell membranes. In this sense G. Ciofani et al., Enhancement of neurite outgrowth in neuronal-like cells following boron nitride nanotube-mediated stimulation, ACS Nano 4, 6267-6277 (2010*)* discloses the use of ultrasound forces to impart mechanical stress to boron nitride nanotubes incubated with neuronal-like PC12 cells. By virtue of their piezoelectric properties, these nanotubes can polarize and convey electrical stimuli to the cells. PC12 stimulated with the method exhibit neurite sprout growth 30% greater than the control cultures after 9 days of treatment. Recently, A. Marino et al., Piezoelectric Nanoparticle-Assisted Wireless Neuronal Stimulation, ACS Nano, in press (2015*)* disclosed tetragonal barium titanate nanoparticles (BTNPs) as nanotransducers whose piezoelectric properties provide indirect electrical stimulation to SH-SY5Y neuron-like cells. Following application of ultrasound to cells treated with BTNPs, fluorescence imaging of ion dynamics revealed that the stimulation was able to elicit a significant cellular response in terms of calcium and sodium fluxes; moreover, tests with appropriate blockers demonstrated that voltage-gated membrane channels were activated; using ultrasounds represents the major drawback of this technique since the area affected thereby is larger than desired, hence losing spatial resolution; besides there are well known side effects derived from the use of the application of ultrasounds, especially in those areas surrounding the cells.

There are some other Noninvasive brain stimulation (NIBS) methods to trigger the action potential of neurons. However, some methods of magnetic stimulation need a conductor element (implanted coil) to induce a current. On the other hand, transcranial magnetic stimulation (TMS) does not require surgery, but suffers from low spatial resolution (1cm). In case of intracranial pulsed ultrasound or low intensity low-frequency ultrasounds (LILFU), some advances have been achieved but still the spatial resolution is about 2 mm. Moreover, the power level used for these methods can have a negative effect in the brain tissue and they need an external instrument to apply the stimulation.

In this patent, the active device has a nanometer size instead of the millimetric active area stimulated by the other methods. Also, there are other important benefits:
- The nanodevices based on ZnO nanostructures can be placed by microinjection (<1µL) on a specific area (e.g. the brain of living mice). Controlling the number of nanodevices in suspension, it should be possible to have extremely fine positioning resolution. The final spatial resolution of activation should be even <10 µm. This is a huge difference compared to other NIBS method. The mechanical or electromagnetic energy to actuate these devices should be sensibly smaller than the one used for direct stimulation for induction or mechanical coupling.
- By functionalizing the nanodevices, they will be attached only on a determined position of the cells. Surfaces functionalized with different neuronal-specific effector molecules, such as cadherin and laminin, can be used to control the adhesion between neurons and nanodevices. Therefore, the spatial resolution can be enhanced and the administration method could be less invasive.
- Putting together these two effects, a nanometric control of the cell stimulation is possible. A controlled amount of nanodevices could be placed in a determined position on specific cell. The electric field generated by the device is local and should not affect to the rest of cells.
- In addition, the electrode arrays or other typical stimulation device can only actuate in a discrete way, because of the size and spacing of the electrodes. They are always working with electrical stimulation discretely distributed. In our case, if a dense solution of nanodevices is applied in a large neural area, a continuous distribution can be get in the whole area, with a different global effect.
- Finally, direct stimulation or enhancement of electrical signals on muscle cells could be achieved because of the electrical-mechanical coupling generated by the nanodevices that will have a close-loop feedback effect.

### DESCRIPTION OF THE INVENTION

In one aspect of the invention, which is defined by claim 1, a self-generating voltage device for cellular electrical activation and stimulation that as a consequence induces cell growth, mobility and/or differentiation is disclosed, said device is a nanoscale piezoelectric voltage-generator device or piezoelectric "nanogenerator" (NG) which is able to modulate the electrical activity of living cells, creating a potential differential locally distributed inside or around the cell. Thus, the interaction of piezoelectric NGs with living cells induces a local electric field in the plasma membrane due to inherent cell forces, which trigger the opening of ion channels present in the membrane allowing spontaneous rapid increases of the intracellular flux of calcium ions. Said device comprises an essentially vertically standing mainly two-dimensional nanostructure, preferably a nanosheet due to its nanometer-scale thickness and high surface-to-volume ratio.

The device of the invention is able to modulate the electrical activity of cells. By creating an electric potential difference locally distributed around the cell due to the presence of an array of nanosheets under the cell culture. Contrary to some of the devices of the art, the device of the invention requires from no external stimuli, such an application of ultrasounds or magnetic fields avoiding the associated drawbacks and side effects.

In a preferred embodiment of the invention the device of the first aspect of the invention may comprise arrays polygonal nanostructures such as ZnO hexagonal nanosheets (NSs) which are used as cell culture substrate cells. It must be understood that even an array of nanosheets (NSs) is meant to render the best possible solution, the aim of the invention may be accomplished implementing just one NS. Said device has a size smaller than a cell, and using the device of the invention the typical value of a cell membrane potential is reduced (<10 mV) being able to generate a potential higher than the typical low value of a cell membrane potential, allowing the instantaneous direct use of the generated electric power to stimulate and grow the cell.

Since the NS is flat, as the skilled in art is aware of flat surfaces being preferred by cells for anchoring and growth, cells will be attached to the surface of the NS. The attachment, anchor, of the cells and the inherent cell forces herein modify the mechanical properties of the NS producing an electric field due to the piezoelectric effect caused by the material of which the NS is made of, and it is well-known that being ZnO a piezoelectric material, it can convert the mechanical stress that a cell can generate into a local DC electric field (dcEF) at the cell membrane.

This means that implementing just one nanosheet may produce the effect of a plurality of any other of the nanostructures known in the art, rendering a solution that does not require from manufacturing lots of CNTs or nanowires in order to achieve the desired effect; as the skilled person, would acknowledge this means a great advantage since less production processes and less resources are needed in order to obtain a high-performance device.

The reduced thickness of less than 100 nm with an aspect ratio higher than 100 makes possible the deflection of these nanostructures due to inherent cell forces. This deflection is translated into the generation of an electric field due to the piezoelectric effect of the ZnO NS. Ultimately, this generated electric field, locally produced nearby the cell plasma membrane, can eventually trigger the opening of ion channels present in the membrane allowing a flux of calcium ions into the cytoplasm. Mechanical stresses developed by cells are typically in the nN range (0.1 nN - 100 nN), would be translated in a piezo potential going from 70 µV to 750 mV, depending on the force magnitude and nanosheet dimensions; the nanosheets of the device invention are essentially flat surfaces which are more prone to be bent than filaments.

In an example, a test flexible unimorph device comprising ZNO or polymer-embedded ZnO nanosheets sandwiched between a gold top electrode and a conducting polyimide substrate is provided, said device generates, after periodically bending the device, voltage peaks that validate the theoretical piezoelectricity of the ZnO nanosheets. In an alternative embodiment of the invention a test flexible bimorph device comprising polymer-embedded ZnO and AIN and metallic contacts, which maybe electrodes or any suitable structure allowing energy supply and/or harvest.

As it is mentioned previously, the interaction of piezoelectric NGs with living cells, since the cells are preferably cultured on top of the NG of the invention, induces a local electric field in their plasma membrane due to inherent cell forces. The *in-situ* electromechanical NG-cell interactions triggered the opening of ion channels present in the cell membrane provoking spontaneous rapid increases of the intracellular calcium levels. Consequently, excellent viability, proliferation and differentiation of cells cultured over the NGs are validated. In this sense, the features that make this biological application suitable for these nanoscale voltage generators are that:
1) They are made of a biocompatible material, thus avoiding any metal diffusion.
2) The NGs of the invention have a size smaller than a cell;
3) The typical cell membrane potential is comparable to the voltage generated by a single NS of the present invention, and
4) The generated electric power is used instantaneously to stimulate the cell for their growing and/or differentiation, without the need of any synergy storage mechanism and without any external stimuli.

As earlier stated, an additional advantage shown by the NGs disclosed in the present invention is that it is biocompatible.

In a possible embodiment of the present invention an implant or a medical device, comprising the NGs or device according to the first aspect of the present invention and also comprising growing and/or differentiating cells on the surface (31) of said device is disclosed.

In an alternative embodiment, the implant or the device of the present invention is a medical device. More preferably, the medical device of the present invention is an implantable or insertable medical device.

As used herein, "medical devices" or "implant" used interchangeably throughout this document can include, for example, stents, grafts, stent-grafts, filters, valves, occludes, markers, mapping devices, therapeutic agent delivery devices, prostheses, pumps, bandages, and other endoluminal and implantable devices that are implanted, acutely or chronically, in the vasculature or other body lumen or cavity at a treatment region.

The medical devices or implant of the present invention can be biocompatible. As used herein, "biocompatible" means suited for and meeting the purpose and requirements of a medical device, used for either long- or short-term implants or for non-implantable applications. Long-term implants are generally defined as devices implanted for more than about 30 days.

Examples of medical devices benefiting from the present invention include implantable or insertable medical devices, for example, selected from stents (including coronary vascular stents, peripheral vascular stents, cerebral, urethral, ureteral, biliary, tracheal, gastrointestinal and esophageal stents), stent coverings, stent grafts, vascular grafts, catheters (e.g., urological or vascular catheters such as balloon catheters and various central venous catheters), guide wires, balloons, filters (e.g., vena cava filters and mesh filters for distil protection devices), abdominal aortic aneurysm (AAA) devices (e.g., AAA stents, AAA grafts), vascular access ports, dialysis ports, embolization devices including cerebral aneurysm filler coils (including Guglilmi detachable coils and metal coils), embolic agents, hermetic sealants, septal defect closure devices, myocardial plugs, patches, pacemakers, lead coatings including coatings for pacemaker leads, defibrillation leads, and coils, ventricular assist devices including left ventricular assist hearts and pumps, total artificial hearts, shunts, valves including heart valves and vascular valves, anastomosis clips and rings, cochlear implants, tissue bulking devices, and tissue engineering scaffolds for cartilage, bone, skin and other in vivo tissue regeneration, sutures, suture anchors, tissue staples and ligating clips at surgical sites, cannulae, metal wire ligatures, urethral slings, hernia "meshes", artificial ligaments, orthopedic prosthesis and dental implants, among others.

The medical devices of the present invention thus include, for example, implantable and insertable medical devices that are used for systemic treatment, as well as those that are used for the localized treatment of any mammalian tissue or organ. Non-limiting examples are tumors; organs including the heart, coronary and peripheral vascular system (referred to overall as "the vasculature"), the urogenital system, including kidneys, bladder, urethra, ureters, prostate, vagina, uterus and ovaries, eyes, ears, spine, nervous system, lungs, trachea, esophagus, intestines, stomach, brain, liver and pancreas, skeletal muscle, smooth muscle, breast, dermal tissue, cartilage, tooth and bone.

In a more preferred embodiment, the medical device of the invention is biocompatible, more preferably by coating with pharmaceutically acceptable polymers and/or functionalized with specific ligands having affinity for a target cell and/or maker molecules that allow tracking thereof.

Additionally, as it is show in the examples includes in the present invention, that the NGs or the medical device of the present invention allow that the generated electric power is used instantaneously to stimulate the cell for their growing and/or differentiation, without the need of any synergy storage mechanism and without any externa stimuli and, more importantly, do not trigger cell cytotoxicity in spite of being growing for long periods under inherent electrical stimulation. It is interesting to note that this *in-situ* cell-scale stimulation can be extrapolate to any of electroconductive cells, such as bone cells, i.e. osteoblast, neurons and/or muscle cells, leading to future bioelectronics medicines based on cell-targeted local electrical impulses in order to electrically self-stimulate cell regeneration, i.e. bone fractures, and increase bone regeneration, treat incipient stages of degerative diseases such as Alzheimer's disease or Amyotrophic Lateral Sclerosis (ALS), rehabilitation for motor disorders or use as distributed pacemaker.

Therefore, the NGs or medical device according to the above-mentioned embodiment of the present invention are utilized in a method *in vitro and*/*or in vivo* for cell activation, stimulation, differentiation or growth promotion and/or regeneration through electrical stimulation.

In a more preferred embodiment thereof, the medical device of the invention is biocompatible, nanodevices comprising one or several NGs and possibly other supporting or additional layers. These nanodevices have been released from the substrate and diluted with a biocompatible solution to form a suspension of biocompatible nanodevices that can be applied to in-vitro or in-vivo cells.

In a preferred embodiment of the second aspect of the invention, aimed to a method of the present invention, the device can be used to assess the effect of electrical signals on the development, differentiation, maturation, functionality and/or survival of electroconductive cells.

For example, one can use the NG or the device of the present invention to provide local electrical signals to the cells to induce them to differentiate along a particular lineage, e.g., to differentiate stem cells into an electroconductive cell type, e.g., neuronal, bone and muscle cells, and subtypes thereof, including cardiomyocytes, osteocytes, skeletal myocytes and the like. Similarly, in some embodiments, one can use the device to provide electrical signals to immature electroconductive cells to enhance their maturation to a more mature phenotype, e.g., by way of an example only, the NG or de medical device of the invention can be used to enhance the maturation of immature osteoblast to mature osteocytes, or immature cardiomyocytes to more mature cardiomyocytes, e.g., with characteristics of mature adult osteocytes or cardiomyocytes found *in vivo.* In a preferred embodiment, the electroconductive cells can be differentiated from cells obtained from a subject, e.g., electroconductive cells derived from stem cells, e.g., induced pluripotent stem cells (iPSC) originally obtained from a subject.

As used herein, "electroconductive cell" refers to a cell being able to conduct, generate, and/or responds to an electrical signal. Non-limiting examples of electroconductive cells can include neurons, osteocytes, monocytes, macrophages, and myocytes (muscle cells). Electroconductive cells can include both naturally-occurring electroconductive cells (e.g., a bone, or muscle cell or neuron) or cells that have been engineered, e.g., genetically modified or transfected to exhibit electroconductive activity. By way of non-limiting example, a cell engineered to express at least one voltage-gated ion channel can be an engineered electroconductive cell. One of skill in the art is familiar with methods for engineering cells, which can include, but are not limited to, genetic modification, homologous recombination, transient expression, and protein transfection and can be accomplished with one or more various vectors, e.g., plasmids, naked DNA, or viral vectors.

In some embodiments, the electroconductive cells can be muscle cells. In some embodiments, the muscle cells can be cardiomyocytes. In some embodiments, the muscle cells can be cardiac pacemaker cells. In some embodiments, the muscle cells can be smooth muscle cells. In some embodiments, the muscle cells can be skeletal muscle cells.

In some embodiments, the electroconductive cells can be neuronal cells. In some embodiments, the neuronal cells can be neuronal cells from the brain, neuronal cells from the spinal cord, dorsal root sensory ganglia, and autonomic ganglia.

In another preferred embodiment, the present invention refers to a culture cell comprising the eletroconductive cells disclosed herein. In some embodiments, the cell culture can further comprise non-electroconductive cells.

Thus, the method for cell activation, stimulation, differentiation, growth promotion and/or regeneration through electrical stimulation disclosed herein comprises the following steps:
a) contact at least an isolated cell with the device (1) disclosed in the present invention,
b) add a culture medium to the at least an isolated cell of step a) which allow that the cell engage the nanostructure (3), preferably nanosheet, of the device (1); and
c) incubate the cell of step b)
wherein the differentiation and/or growing of the cells induce an electrical stimulus through the mechanical stress produced in the device (1) and wherein the method it is characterized in that there is not any external stimuli.

In a preferred embodiment, cells can be cultured more naturally, enabling the cultured cells and tissue to be studied, but in a more natural context. As it is mentioned previously, the cells are cultured in contact with the device (1), preferably on the surface of the NGs or the medical device. In accordance with some embodiments of the invention, the NGs or the medical device include a cell culture surface upon which the cells and tissue to be studied can be cultured. The cell culture surface preferably has a nanometer sized features that encourage the cells and tissue to culture in configurations that more closely model the way the cells and tissues would develop in the body, such as when using an extracellular matrix.

In a preferred embodiment, the method disclosed herein it is characterized in that the cell is an electroconductive cell.

With regard to the method of the present invention, the NGs or the medical device used in the method facilitates the interaction of piezoelectric NGs with living electroconducieve cells induces a local electric field in the plasma membrane due to inherent cell forces, which trigger the opening of ion channels present in the membrane allowing spontaneous rapid increases of the intracellular flux of calcium ions. The intracellular flux of calcium ions modulates the cell stimulation and the possibility of improving the conditions of cultured tissues in terms of metabolism, proliferation, extracellular matrix production and metabolite production. In fact, on several cell typologies electrical stimulation has long been proved to have positive effects on their growth. The solution represented by the invention allows achieving these results with no need of external circuits for stimulation, electrical connections or other devices connected to the cultures.

In a preferred embodiment of the method, the NG or the medical device of the invention is preferably biocompatible and/or functionalized, more preferably it is biocompatible and functionalized with specific ligands or with marker molecules. The NGs or the medical device used in the method of the present invention are stably and homogeneously dispersed in the culture medium, in concentrations not entailing toxic effects for the cultured cell.

Functionalization in the sense of the present invention is understood to refer in general to measures as a consequence of which the NGs or medical devices of the present invention gains additional functions. Functionalization, according to this invention, comprises the incorporation or attachment of substances to the surface of the NGs or medical device of the invention. Suitable substances are selected from pharmacological active ingredients, linkers, microorganisms, cells of plant or animal origin including human cells or cell cultures and tissue, minerals, salts, metals, synthetic or natural polymers, proteins, peptides, amino acids, solvents, etc.

The NGs or medical devices disclosed herein can be functionalized with different neuronal-specific effector molecules, such as cadherin and laminin, to control the adhesion with neurons. Therefore, the spatial resolution of the electrical stimulation can be enhanced.

The NGs or medical devices can be functionalized by making it more biocompatible before or after a possible loading with active ingredients. This is done by coating it with at least one additional layer of biodegradable and/or absorbable polymers such as collagen, albumin, gelatin, hyaluronic acid, starch, celluloses such as methyl cellulose hydroxypropylmethyl cellulose, carboxymethyl cellulose phthalate; casein, dextrans, polysaccharides, fibrinogen, poly(D,L-lactides), poly(D,L-lactide-co-glycolides), poly(glycolides), poly(hydroxybutylates), poly(alkyl carbonates), poly(orthoesters), polyesters, poly(hydroxyvaleric acid), polydioxanones, poly(ethylene terephtalate), poly(malic acid), poly(tartronic acid), polyanhydrides, polyphosphazenes, poly(amino acids) and their copolymers or non-biodegradable and/or absorbable polymers. In particular, anionic, cationic or amphoteric coatings are especially preferred, such as alginate, carrageenan, carboxymethyl cellulose, chitosan, poly-L-lysine and/or phosphorylcholine. Also, dielectric layers such as silicon dioxide, silicon nitride, SU8 or other biocompatible passivation layers can be utilized.

Another aspect, however not forming part of the invention , refers to a second method for repairing an injury or damaged tissue in a subject, wherein the method comprises the method of the invention described above and additionally step (d) as follows:
a) contacting at least an isolated cell with the device (1) of the invention,
b) adding a culture medium to the isolated cell of step a) which allows that the cell engage the nanostructure (3), preferably nanosheet, of the device (1),
c) incubating the cell of step b), and
d) implanting the device of step c) in the subject,
wherein, as described above, the differentiation and/or growing of the cells in step (b) induce an electrical stimulus through the mechanical stress produced in the device (1) and wherein the method is characterized in that there is not any external stimuli.

In the second method, the tissue is selected from the list consisting of: muscle, nervous, bone, cartilaginous, myocardial tissues, tendons and ligaments.

More preferably, the device implanted in step (d) is biocompatible, as described above, by coating with pharmaceutically acceptable polymers and/or functionalized with specific ligands having affinity for a target cell and/or maker molecules that allow tracking thereof.

The term "cell culture medium" (also referred to herein as a "culture medium" or "medium") as referred to herein is a medium for culturing cells containing nutrients that maintain cell viability and support proliferation. The cell culture medium may contain any of the following in an appropriate combination: salt(s), buffer(s), amino acids, glucose or other sugar(s), antibiotics, serum or serum replacement, and other components such as peptide growth factors, etc. Cell culture media ordinarily used for particular cell types are known to those skilled in the art.

The term "differentiation" as referred to herein refers to the process whereby a cell moves further down the developmental pathway and begins expressing markers and phenotypic characteristics known to be associated with a cell that are more specialized and closer to becoming terminally differentiated cells. The pathway along which cells progress from a less committed cell to a cell that is increasingly committed to a particular cell type, and eventually to a terminally differentiated cell is referred to as progressive differentiation or progressive commitment. Cell which are more specialized (e.g., have begun to progress along a path of progressive differentiation) but not yet terminally differentiated are referred to as partially differentiated. Differentiation is a developmental process whereby cells assume a more specialized phenotype, e.g., acquire one or more characteristics or functions distinct from other cell types. In some cases, the differentiated phenotype refers to a cell phenotype that is at the mature endpoint in some developmental pathway (a so called terminally differentiated cell). In many, but not all tissues, the process of differentiation is coupled with exit from the cell cycle. In these cases, the terminally differentiated cells lose or greatly restrict their capacity to proliferate. However, in the context of this specification, the terms "differentiation" or "differentiated" refer to cells that are more specialized in their fate or function than at one time in their development. A cell that is "differentiated" relative to a progenitor cell has one or more phenotypic differences relative to that progenitor cell and characteristic of a more mature or specialized cell type. Phenotypic differences include, but are not limited to morphologic differences and differences in gene expression and biological activity, including not only the presence or absence of an expressed marker, but also differences in the amount of a marker and differences in the co-expression patterns of a set of markers.

As used herein, "proliferating" and "proliferation" refers to an increase in the number of cells in a population (growth) by means of cell division. Cell proliferation is generally understood to result from the coordinated activation of multiple signal transduction pathways in response to the environment, including growth factors and other mitogens. Cell proliferation may also be promoted by release from the actions of intra- or extracellular signals and mechanisms that block or negatively affect cell proliferation.

As used herein, the terms "treat," "treatment," "treating," or "amelioration" refer to therapeutic treatments, wherein the object is to reverse, alleviate, ameliorate, inhibit, slow down or stop the progression or severity of a condition associated with a disease or disorder. The term "treating" includes reducing or alleviating at least one adverse effect or symptom of a condition, disease or disorder. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

The method for electrical stimulation of cells by the NGs or medical device of the present invention can find numberless applications in the biomedical field, both clinical and pre-clinical, such as gastric stimulation following gastroparesis, cardiac stimulation, neural stimulation, muscle stimulation. With regard to clinical applications, deep brain stimulation is a treatment of proven effectiveness for high-impact pathologies such as Parkinson's disease, chronic tremor, dystonia and other hyperkinetic disorders.

In addition, cell stimulation finds wide use in regenerative medicine and/or tissue engineering applications. This technique has high potential for use as a novel method for rehabilitation of patients having muscle denervations of various origins. Moreover, this method allows to improve the conditions of cultivated tissues in terms of metabolism, proliferation and production of extracellular matrix.

Exemplary tissues susceptible of being treated in accordance with the present invention are the muscle, nervous, bone, cartilaginous, myocardial tissues, or any other tissue or organ, such as tendons and ligaments, requiring a regenerative or reconstructive treatment or an acute, chronic, neuromuscular pain treatment, or a healing treatment of damaged tissues. Specific cell types whose differentiation and growth or proliferation is activated, stimulated or promoted by electrical stimulation with NGs or medical devices comprise muscle cells, myoblasts, neural cells, myocardial cells, osteoblasts, osteoclasts, cardiac stem cells, induced pluripotent stem cells, stem cells in general and the any electroconductive cells known.

In some embodiments, the cell cultured in contact with the NG or medical device of the invention system can comprise a plurality of electroconductive cells. In some embodiments, the plurality of cells can form a monolayer of cells. In some embodiments, the plurality of cells can form a tissue, e.g. a muscle tissue, bone tissue or nerve tissue. In some embodiments, the cell cultured can further comprise non-electroconductive cells, e.g., fat cells, endothelial cells, or epithelial cells.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:

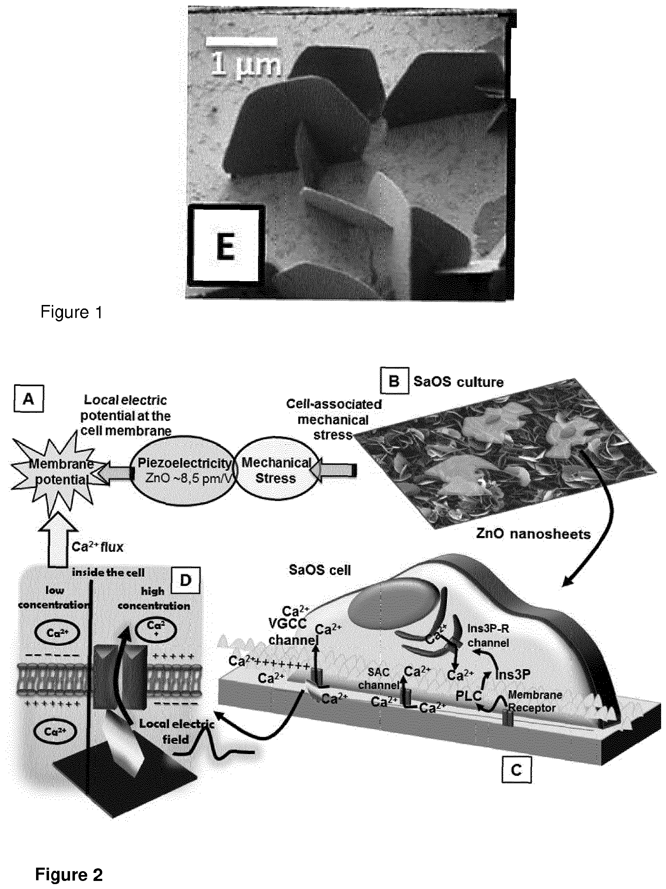

Figure 1. - Shows a SEM image where a plurality of nanosheets may be appreciated.
Figures 2a-2d.- Show a diagram depicting the working principle of the invention (fig .2a) cell culture on top of ZnO NSs(fig .2), the effect of the nanogenerators in the cell membrane and the calcium flux (fig .2c) and graphical explanation of the working principle of a voltage dependent calcium channel (VDCC) (fig .2d).

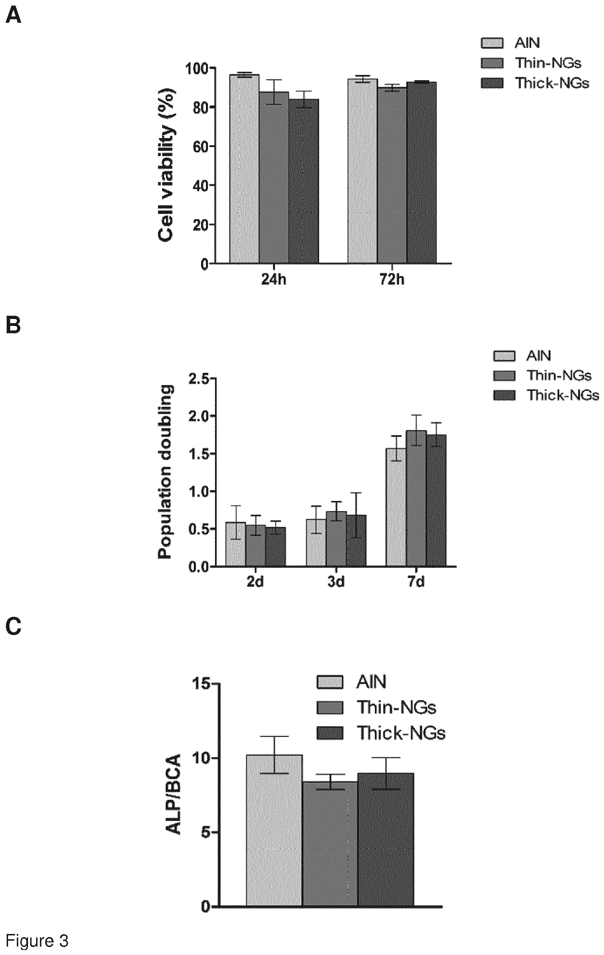

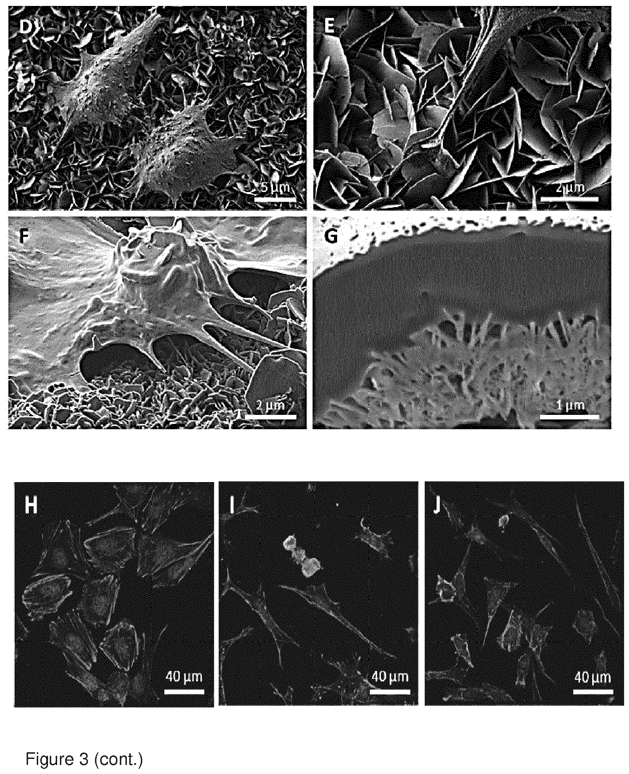

Figures 3 A-J - Saos -2 cells grown on the thin ZnO-NGs (thin-NG) thick-NG and AIN (as control). Biocompatibility of materials were analyzed by **(A)** live/dead kit al 24 and 72 h of culture and no differences were found in cell viability among materials an time-points analysed. **(B)** Alamar Blue Assay at 1, 2, 3 and 7 days with no differences among materials and time-points analyzed and **(C)** quantitative analysis of ALP activity, a differentiation osteoblast marker, at 14 days of culture with no significant differences among materials. Data are mean±SD, n=3 independent experiments. *p>0.05 from _{χ}2 test and Kruskal-Wallis test. Cell adhesion and spreading over AIN (D), thin-NG (E) and thick-NG (F) were analyzed by immunodetection of vinculin (focal contacts, green) and detection of actin filaments (stress fibers, red). Morphology and NG-cell interaction was assessed by scanning electron microscope and focus ion beam (FIB); (G) cells were firmly adhered to NGs, (H) with long prolongations ending at the NSs. (H) Cells were completely adapted to the topography of the NSs (I) which were in intimate contact with the plasma membrane.

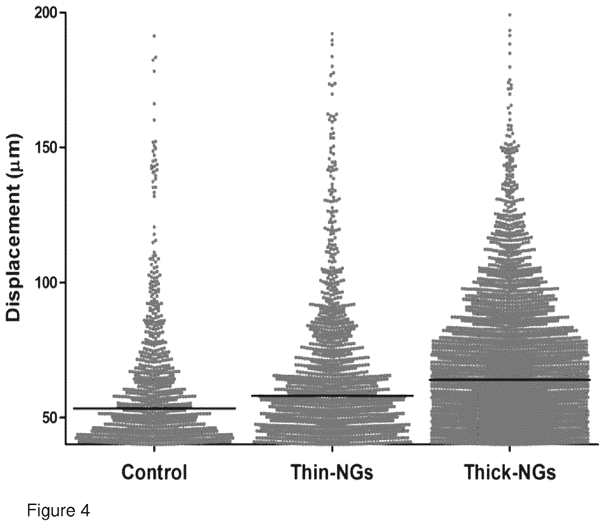

Figure 4. Quantification and distribution of macrophage motility on materials tested. Motility was quantified as the trajectory length of each cell, distances shorter than 40 µm were considered in-situ motions and not displacements. Significant difference was found in the median (black line) distance length, being the macrophages grown on thick-NG the more active compared with the other two materials. Macrophages grown on thin-NG were also more active than the ones grown on glass. Data are mean±SD, n= 3 independent experiments. *P≤ 0.05 from Kruskal-Wallis test

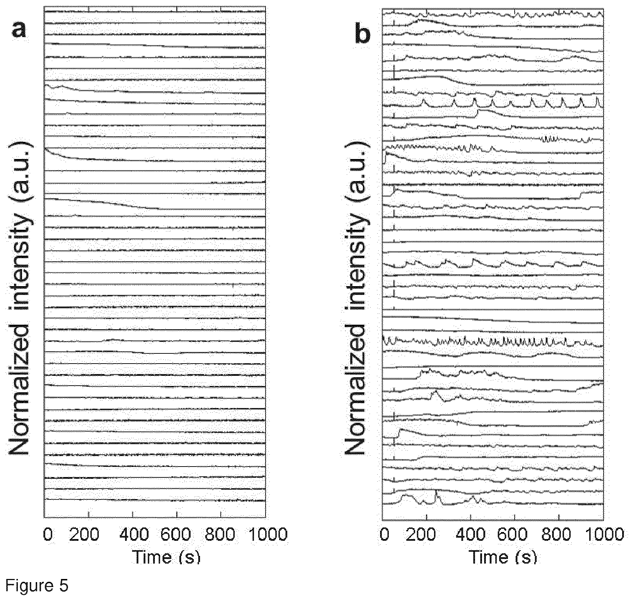

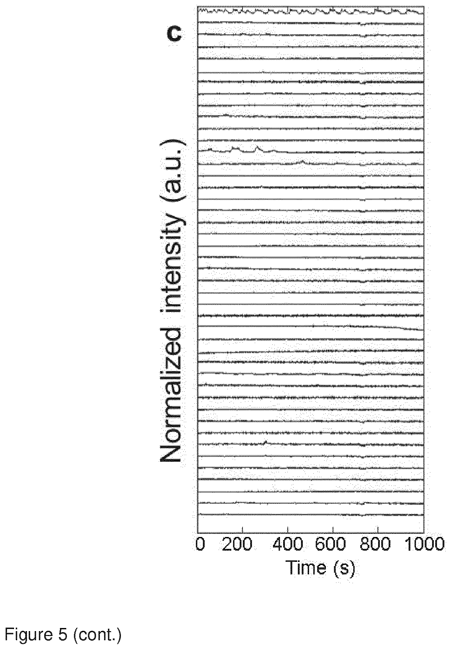

Figure 5. Fluo-4 AM fluorescence reflecting increases of the intracellular Ca²⁺ concentration in Saos-2 cells versus time. a) The number of Saos-2 cells presenting changes in their Ca²⁺ concentrations is very low when cultured over glass coverslip. b) By contrast, a high number of cells present changes in its calcium concentration when cultured over thin-NGs. Besides, the amplitude of the Ca²⁺ influx and the duration of the influx vary from cell to cell. c) Finally, several osteoblast present changes in their Ca²⁺ concentration, but the amplitude of these Ca²⁺ increase is in general low. Each curve corresponds to the relative intensity normalized to the mean value of the brightness in every cell, which is directly related to the changes of Ca²⁺ concentration. The graph axis ranges have been limited to 45 cells and 1000 s.

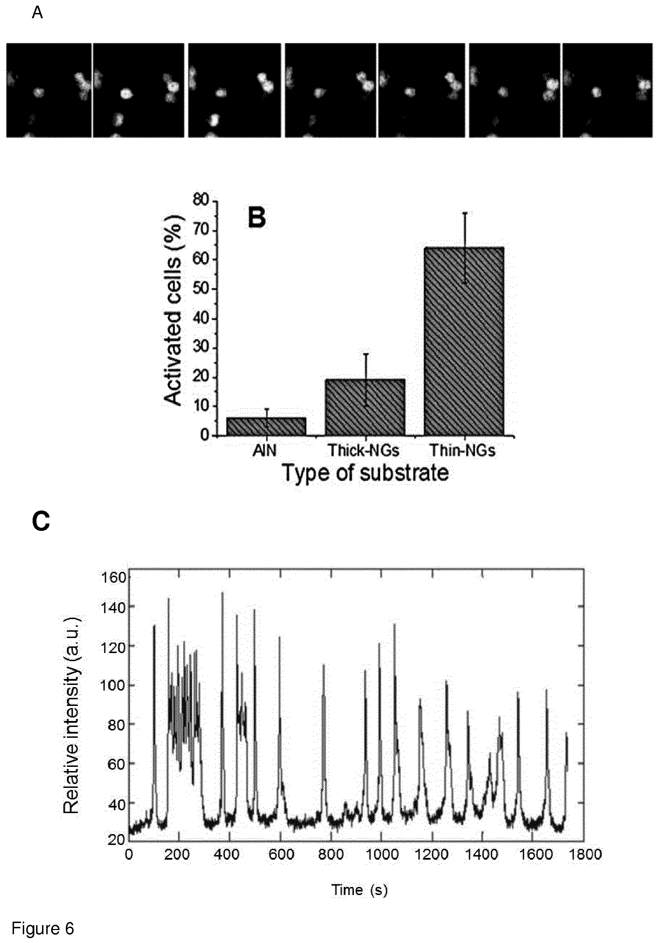

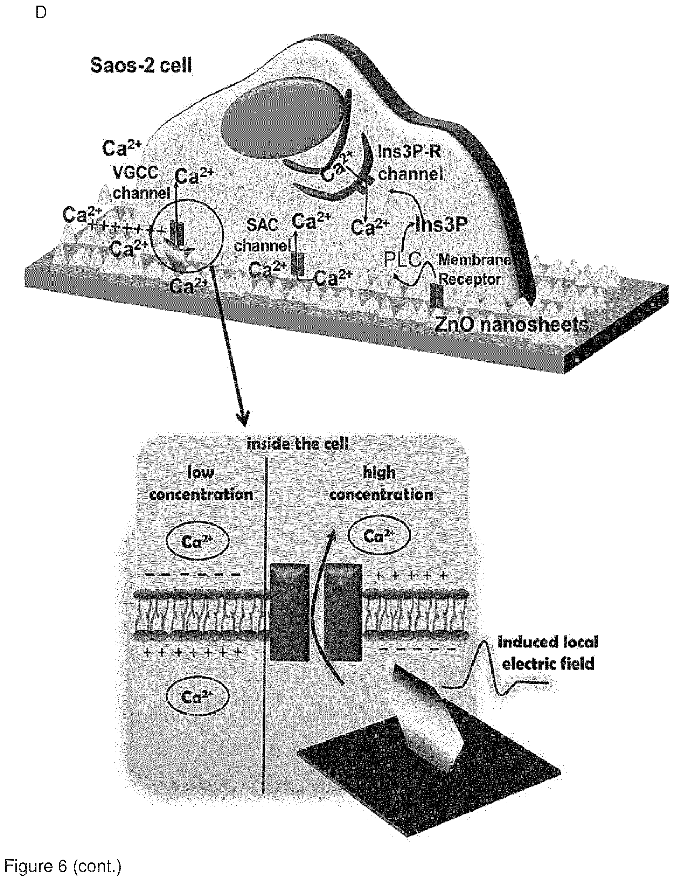

Figure 6. Effect of thin- and thick-NGs. (A) Changes in Ca²⁺ concentration in Saos-2 cells analyzed by Fluo-4 AM and recorded every 1 sec during 30 min using time-lapse confocal microscopy. (B) Quantification of cells undergoing changes in Ca²⁺ concentration; the number of cells activated was significantly higher in thin-NG than in thick-NG and glass material, as it was the number of cells activated in thick-NG compared to glass. Data are mean±SD, n= 3 independent experiments. *P≤0.05 from χ2 test. (C) Ca²⁺ influxes pattern of a selected cell grown on thin-NGs. (D) Sketch of a cell grown on top of a NG microarray indicating the possible pathways involved changes in the Ca²⁺ concentration. Extracellular Ca²⁺ influx is due to the opening of plasma membrane channels, either voltage gathered Ca²⁺ (VGC) or stretch-activated ion (SAC) channels, whereas intracellular Ca²⁺ comes from endoplasmic reticulum storage through the activation of membrane receptors. The bending of an NG will induce a local electrical that will create a potential difference around the plasma membrane that will provoke the opening of the Ca²⁺ channels.

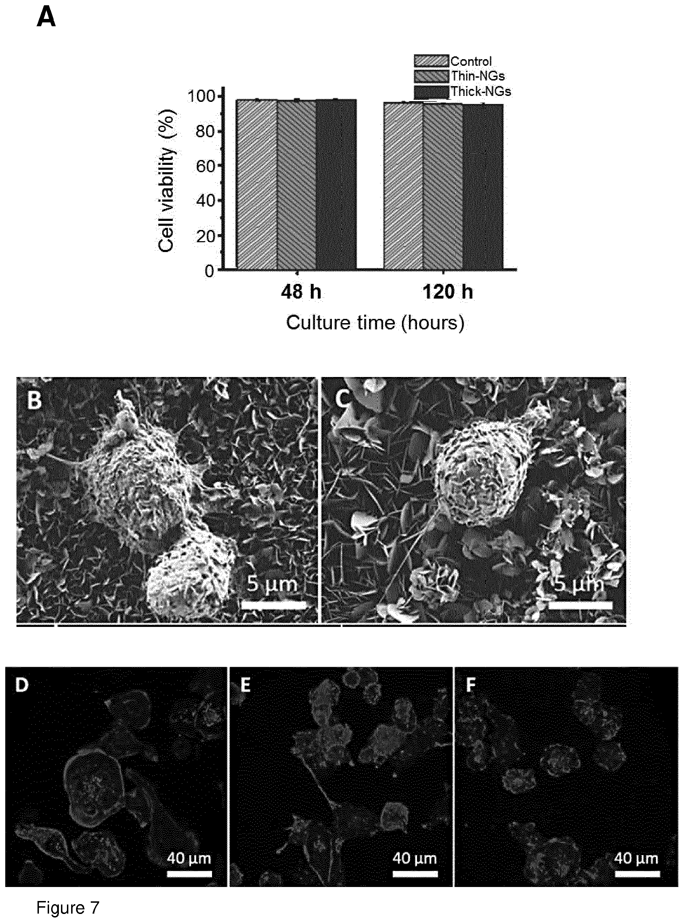

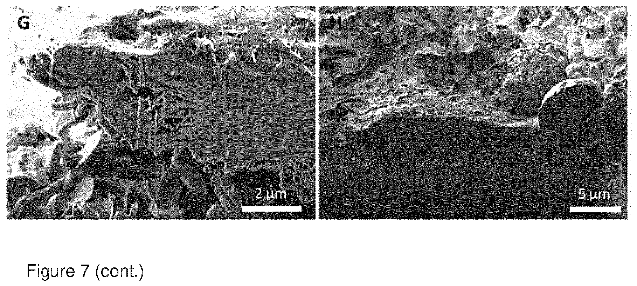

Figure 7. Macrophages viability on thin-NGs, thick-NGs and AIN (as control material). Cytotoxicity was analyzed by live/dead kit at 2 and 5 days of culture. No differences were found among samples and time-points analyzed (A). Data are mean±SD, n= 3 independent experiments, and statistical significance was considered when P < 0.05 from χ2 test. Cell morphology after 2 days in culture was assessed by scanning electron microscopy on thin-NGs (B) and thick-NGs (C). Stress fibers distribution and spreading were analyzed by the detection of actin filaments (red). Cells adhered to the materials presented similar stress fibers distributions on AIN (D), thin-NGs (E) and thick-NGs. NGs-cell interaction was assessed by scanning electron microscope and focus ion beam. Cells were adhered to thick-NGs, with macrophages surrounding the NGs (G) and macrophages covering the NGs topography (H).

### PREFERRED EMBODIMENTS OF THE INVENTION

In a preferred embodiment of the first aspect of the invention, namely a device (1) for electrical living cell stimulation (11) hereinafter NG [Nanogenerator], the device comprises at least one nanostructure (3) grown on a substrate (2) made of one of: silicon, polysilicon, silicon dioxide, glass, SU8, AIN, or Aluminum oxide and metals, said nanostructure (3) being made of a piezoelectric material and preferably grown on said substrate (2). Said nanostructure (3) is preferably made of ZnO and essentially flat, preferably a ZnO nanosheet (3) with a constant or variable thickness comprised between T1-T2. The nanostructure (3) may show different geometries and shapes, preferably polygonal shapes; although hexagon or a trapeze shapes are more preferred.

The nanosheet (3) may be disposed vertically to the substrate (2), preferably orthogonally, the width of the nanosheet (3) may be constant or may vary (increase or decrease) along the nanosheet (3). In an alternative embodiment, the nanostructure (3) may have different thicknesses or cross section, said nanostructure (3) being a nanosheet (3). Preferably, the nanostructure (3) is a nanosheet (3) with a high aspect ratio, *i.e.* it is thin compared to its width.

In order to produce the at least one nanosheet (3) of the device as the one shown in figure 1, different procedures may be carried out; ZnO nanosheets (3) may be grown through a hydrothermal chemical method.

For the purpose of the invention herby described nanosheets (3) are preferably produced by hydrothermal synthesis of ZnO nanosheets (3) due to its simplicity and environmentally friendly conditions. The resulting nanosheets (3) showed high crystallinity quality, good uniformity and growth reproducibility (Fig. 1).

In order to study the dependence on the nanosheets (3) aspect ratio and the generated electric field strengths by the device (1) - cell (11) interaction, two different nanosheets (3) morphologies were synthesized. Hereinafter we use the term 'thin nanogenerator' (thin-NG) to refer a thin device (1) with nanosheets (3) with a thickness of 20 ± 1.34 nm and 'thick nanogenerator (thick-NG) to refer a thick device (1) with nanosheets (3) with a thickness of 40 ± 4.5 nm and a mean diameter of 1.34 ± 0.09 µm and 3.08 ± 0.82 µm, respectively (Fig. 1, B and C). It has been calculated a higher generated piezopotential in thin devices (1) respect to thick devices (1) for the same cell force (Fig. 1D). Mechanical stresses developed by cells (11), which are typically in the nN range (0.1 nN - 100 nN), (6, 7) would be translated in a piezopotential going from 70 µV to 750 mV, depending on the force magnitude and nanosheets (3) dimensions.

To test the device (1) direct piezoelectric effect, a flexible unimorph device (1) was fabricated, said flexible unimorph device (1) comprising polymer-embedded ZnO nanosheets (3) sandwiched between a gold top electrode and a conducting polyimide substrate. The voltage peaks generated after periodically bending the test device (1) validated the theoretical piezoelectricity of the ZnO nanosheets (3).

The nanostructure (3) produced is a nanosheet (3) that has an aspect ratio higher than 100 and is essentially flat so that the cell (11) may anchor on the surface (31) of the nanostructure (3) so that a mechanical stress is produced in the nanostructure (3) generating a voltage due to the piezoelectric character of the nanostructure (3), this generated electric field, locally produced nearby the cell plasma membrane, triggers the opening of ion channels present in the membrane allowing a flux of calcium ions into the cytoplasm; thus promoting cell growth as depicted in figure 2a.

The NG of the first aspect of the invention was used to evaluate the NG-cell interaction using two different human cell lines, Saos-2 (osteoblast-like cells) and monocytes THP1 which were differentiated to macrophages.

Human THP-1 monocyte cells were grown under standard conditions (37 °C and 5% CO2) in RPMI 1640 medium (Life Technologies) supplemented with 25% fetal bovine serum (FBS, Life Technologies) and 5% L-glutamine (Biowest). To differentiate monocytes into macrophages, cells were treated with 0.16 µM phorbol-12-myristate-13-acetate (PMA, Sigma) for 24, 48 or 120 h depending on the assay performed. Human osteosarcoma Saos-2 cells (ATCC) was cultured in Dulbecco's modified Eagle medium (DMEM) (Invitrogen) with 10% FBS under standard conditions.

Saos-2 cells present voltage-gated calcium channels (VGCCs) and stretch-activated cation channels (SACCs) that can be activated electrically. In addition, it is known that electrical stimulation of bone fractures increases cell proliferation, mineralization of cell matrix and synthesis of proteins characteristics of osteoblast differentiation. To evaluate the feasibility of using the NG of the present invention as electrical stimulator of living cells, the inventor first assessed the biocompatibility of the thin- and thick-NG of the present invention, using AIN thin-film and glass coverslip as control substrates.

Briefly, the samples (AIN, thin-NGs and thick-NGs) were sterilized with absolute ethanol and individually introduced into a 4-well plate. For osteoblast studies, 50,000 cells were seeded into each well and cultured under standard conditions for 24 and 72 h, whereas for macrophage analysis, 100,000 monocytes were seeded into each well and differentiate into macrophages for 48 and 120 h. In parallel, control cells were seeded directly onto a glass coverslip in the absence of samples. Cytotoxicity was analyzed using the Live/Dead Viability/Cytotoxicity kit for mammalian cells (Invitrogen), according to the manufacturer's protocol. Live cells with intracellular esterase activity show green fluorescence, whereas dead cells show red fluorescence because of the permeability of their damaged plasma membrane to the ethidium homodimer. Cultures were observed under an Olympus IX71 inverted microscope equipped with epifluorescence. Images from different regions were captured, and a minimum of 300 cells were analyzed. All experiments were performed in triplicate.

Later, it was analyzed the morphology and adhesion on the NG of the invention of Saos-2 through the visualization of the local contact, which transmit the intracellular tension to the underlying substrate. The same samples used for the viability assay were processed to be analyzed by scanning electron microscopy (SEM) and focused ion beam (FIB). Cells were washed in phosphate buffered saline (PBS), fixed in 4 % paraformaldehyde in PBS for 15 min at RT and washed again in PBS. Cell dehydration was performed in a series of increasing ethanol concentrations (50, 70, 90 and twice 100 %) for 8 min each. Finally, samples were dried using hexamethyldisilazane (HMDS; Electron Microscope Science) for 15 min. Samples were mounted on special stubs and analyzed using a SEM (Zeiss Merlin) in order to observe cell morphology. In addition, samples were cut using a FIB in order to observe the interaction between cells and piezoelectric material.

Cytoskeleton organization and focal contacts were determined by actin filaments and vinculin detection. Following the same protocol described for the viability assay, cells were seeded onto samples and, after 24 h in the case of osteoblasts and 48 h for macrophages, cells were fixed in 4 % paraformaldehyde in PBS for 15 min at RT. Then, cells were permeabilized with 0.1% Triton X-100 (Sigma) in PBS for 15 min and blocked for 25 min with 1% bovine serum albumin (BSA; Sigma) in PBS at RT. Samples were then incubated with a mouse anti-vinculin primary antibody (Chemicon) for 60 min at RT and washed with 1% BSA-PBS. Then, samples were incubated with a mixture of Texas Red-conjugated phalloidin (Invitrogen), Alexa fluor 488 goat anti-mouse IgG1 and Hoechst 33258 (both from Sigma) for 60 min at RT. Finally, cells were washed in PBS, air dried and mounted on a specific bottom glass dishes (MatTek) using ProLongAntifade mounting solution (Life Technologies). Actin cytoskeleton evaluation was done in a confocal laser scanning microscope (CLSM, Olympus).

Osteoblasts proliferation was determined using Alamar Blue cell viability reagent (Invitrogen). 250,000 Saos-2 cells were seeded into each well of a 4-multiwell plate containing each sample type. After 24 h, samples with adhered cells were moved to a new 4-multiwell plate containing fresh medium with 10% Alamar Blue. After 4 h in standard conditions supernatant was withdrawn and its fluorescence quantified using a fluorimeter. Fresh medium was added to the cultures and the assay was repeated after 72 h and 7 days. Experiments were performed in triplicate.

Saos-2 differentiation onto the sample surfaces was analysed by quantifying the alkaline phosphatase (ALP) activity, considered an early stage marker of osteoblast differentiation. Thus, 500,000 cells were seeded into 35 mm culture dishes containing a pre-sterilized sample. After 14 days in culture replacing the medium replaced every 3-4 days, ALP activity was measured. Briefly, each sample was transferred to an Eppendorf tube and cells were lysed using 2x CyQuant cell lysis buffer (Invitrogen) for 10 min and vortexed for 15 s. Cell lysates were centrifuged at 12,000 rpm for 4 min at 4°C and supernatants were collected. ALP activity was evaluated quantifying the p-nitrophenol (pNP) produced by the hydrolysis of pNP phosphate (pNPP; ThermoScientific), according to the manufacturer's protocol. The absorbance was measured at 405 nm using NanodropSpectrphotometer (ThermoScientific). ALP activity was normalized to total protein content using the Micro BCA Protein Assay kit (ThermoScientific).

No differences in cell viability, proliferation and differentiation were observed between both thin- and thick-NGs of the present invention and between them and controls (AIN) at different time-points analyzed (Fig. 3A-C). These results show that NGs do not interfere with these cell activities in contrast to ZnO nanoflowers (Park JK. et al., Adv Mater. 2010; 22:4857-4861) or nanorods (Lee J. et al., Biomaterials. 2008; 29:3743-9), where a decrease of proliferation was observed when compared to control substrates. The obtained results show that Saos-2 cells adapt their shape to the topography being in close contact to the NS network (Figure 3 G-J); short and long cell prolongations were observed firmly attached to individual NSs. Afterwards, the inventors analyzed the Saos-2 adhesion on the NGs of the invention through the visualization of the focal contacts, which transmit the intracellular tension to the underlying substrate. The distribution of focal contacts through vinculin detection, a cytosolic protein involved in the adhesion, and actin stress fibers, were compared among the Saos-2 cells grown on the materials analyzed. Saos-2 cells showed slight differences in the distribution and number of focal contacts among the three substrates analyzed (Fig 3 D-F). Cells grown on flat AIN substrate were well spread with a high number of focal contacts at the end of the actin stress fibers, which were well defined and running in parallel crossing the cell from end to end (Fig 3H). By contrast, Saos-2 cells adhered to the thin- or thick-NGs of the present invention presented fewer stress fibers compared to the AIN substrate, running in parallel but with a disrupted aspect, being the number of focal contacts also inferior (Fig 3I and 3F). As cells were adapted to the topography of the NSs, stress fibers could not cross the cell as lineal and parallel bundles because cells were not able to attach wherever, but only in the surfaces of the NSs (Fig 3H and 31). It was easier to adhere to the thin-NGs were discontinuities were lower than to thick-NGs where the sheets were more separated. These differences were also observed in SEM images. Whereas on AIN, the cells showed a polygonal shape, on thin-NG showed a preferentially spindle shape and on thick-NG a mixture of both morphologies were present.

The intracellular tension transmitted via focal contacts generates a force on the NGs of the invention that result in a local electric potential difference around the cell plasma membrane. It is well known that osteoblasts exposed to an electrical field can undergo changes in its calcium concentration ([Ca²⁺]). In this sense, changes in [Ca²⁺] can be recorded and quantified in cells electrically stimulated using the Fluo-4 AM dye.

Accordingly, osteoblasts loaded with this dye and grown over thin- or thick-NGs disclosed in the present invention and glass coverslip were recorded every 1 sec for 30 min using time-lapse confocal laser scanning microscopy. Thus, time-lapse CLSM (Leica SP5) was used to measure the intracellular calcium increase over time. Saos-2 cells were cultured on samples surface for 24 h in standard conditions, then cells were loaded with 2 µM Fluo-4 AM and 0.02% pluronic acid (both from Life Technologies) in serum free DMEM for 30 min in the dark at RT. Samples were washed with serum free DMEM and then transferred to MatTek dishes with fresh medium. Images of osteoblasts were captured in time-lapse CLSM every 1 sec during 30 min. Changes in fluorescence intensity during the time of monitoring were processed using Image J software. A MATLAB code has been developed to automatically detect Ca²⁺ increase in cells, taking the time-lapse movies recorded in the CLSM as data source. Several image enhancement routines and perimeter detection algorithm has been used to detect all the cultured cells. Then, mean relative intensity along time has been calculated for each particular cell, using the automatic suitable threshold for every different measurement. Finally, relative intensity corresponding to each single cell (Fig. 4 and 5) was used as input of an ad-hoc peak detector to evaluate whether the cell was activated by the NGs.

A 64±19% of osteoblast grown over thin-NGs experimented increases in [Ca²⁺] (Fig 6A and B) with amplitudes of Ca²⁺ transients (Fig 6C) compatible with an influx of extracellular calcium. By contrast, the percentage of osteoblast grown over thick-NGs that presented increases in [Ca²⁺] was only 19±9% (Fig 6B), with low amplitudes of Ca²⁺ transients (Fig 7) and this percentage dropped to 6±3% (Fig 4B, 6) in osteoblast grown over glass coverslips. These results show that inherent osteoblast adhesion forces are able to bend thin-NGs but in lesser extend thick-NGs due to their geometry differences and subsequently elastic constants. The number of cells grown over glass coverslips undergoing an increase on [Ca²⁺] was nearly negligible, thus, reinforcing that the interaction of piezoelectric NGs with living cells induces a local electric field which is high enough to activate their ion-channels.

Additionally, the present invention demonstrates that each NS in contact to the cell membrane induce a local electric field (Fig 6D) that can locally change the membrane potential and trigger the opening of VGCC or SACC. These channels allow an influx of extracellular Ca²⁺ that will produce high amplitudes of Ca²⁺ transients. Moreover, electrical stimulation can reorganize plasma membrane proteins that are coupled via phospholipase C (PLC) to the release of intracellular Ca²⁺ storage translated to low amplitudes of Ca²⁺ transients. The different peaks of Ca²⁺ registered (Fig 6B and 5) corresponding to the interaction between osteoblasts and thin-NGs, that is, low-, medium or high-amplitude, and short, medium or long duration of Ca²⁺ transients (Fig 6B) are due to the combination of the three different mechanisms working together. The mechanical stress produced by a single osteoblast could be different for each cell motion and adhesion, depending of the length of the cell prolongation emitted and the intensity of the strength necessary to adhere to a single NS. Thus, each movement will produce a different local voltage and depending of its intensity, the mechanisms induced to increase [Ca²⁺] could be different.

Macrophages are phagocytic cells involved in the immunological response. No significant differences were found in macrophage viability when grown on the different NGs analyzed in the present invention. In all cases, viability was superior to 95% at 48 h and 5 days (Fig 7A). The distribution of focal contacts and actin stress fibers was similar in macrophages adhered to the NGs or to the control materials (Figure 7D; E and F), as it was the morphology of the cells visualized under the scanning electron microscope (Figure 7B and C). Macrophages grown on ZnO-NGs were transversally cut using the focus ion beam (FIB) and the plasma membrane was conformably covering the NSs substrate topology, being both in close contact (Fig 7G and H). Thus, NGs were biocompatible and did not disturb the adhesion of macrophages to its surface up to 5 days in culture.

Furthermore, the inventors analyzed the effect of the NG on the capacity of macrophages to migrate recording the motility of macrophages grown on thin- or thick-NGs and glass (as control) every 5 min and during 4 h using time-lapse confocal microscopy CLSM (Leica SP5). Motility was evaluated as the trajectory length of each cell. Distances shorter than 40 µm were considered in-situ motions, and were not taken into account. Macrophage cultures were incubated with the CellTracker green CMFDA (Life Technologies), according to manufacturer's protocol. Samples with attached cells were transferred to MatTek dishes containing fresh medium and images from different regions were captured in 20 different z-stacks every 5 min for 4 h using a 10x objective. Captured images were analyzed using Imaris software (Bitplane) to determine cells track length.

Macrophages migrated in all materials tested, but higher trajectories length on thick-NG > thin-NG > thin-NG > glass material, being the differences significant (Fig 4). The inventors do not apply a direct current, but macrophage motility could have bent the NSs and created an electrical field that would in turn activated the macrophages, and/or the topography of the NGs could have provoked differences in macrophage motility. The combination of both parameters, topography and direct electrical field, is the cause that activity was higher on thick-NGs.

The above described embodiments are given as illustrative examples only. It will be readily appreciated that many deviations may be made from the specific embodiments disclosed in this specification without departing from the scope of the invention. Accordingly, the scope of the invention is to be determined by the claims below rather than being limited to the specifically descried embodiments above.

## Claims

1. Self-generating voltage device (1) for electrical stimulation of cells (11), preferably for the cell differentiation, mobility and/or growth, the device comprising at least one nanostructure (3) made from a piezoelectric material, wherein the nanostructure (3) has an aspect ratio higher than 100 and is essentially flat so that the cell (11) may anchor on the surface (31) of the nanostructure (3) so that a mechanical stress is produced in the nanostructure (3) generating a voltage due to the piezoelectric character of the nanostructure (3), the device being **characterized in that** the width of the nanostructure (3) decreases along the nanostructure (3).

2. Self-generating voltage device (1), according to claim 1 **characterized by** the nanostructure (3) being made of: ZNO, or ZNO+AINor polymer-embedded ZnO and AIN.

3. Self-generating voltage device (1), according to any of claims 1 to 2 **characterized in that** the nanostructure (3) has a thickness comprised between 20 ± 1.34 nm and 40 ± 4.5 nm.

4. Self-generating voltage device (1), according to any of claims 1 to 3 wherein the nanostructure (3) has a maximum thickness of 100nm.

5. Self-generating voltage device (1), according to any of claims 1 to 4 **characterized in that** the nanostructure (3) has a constant thickness.

6. Self-generating voltage device (1), according to any of claims 1 to 5 **characterized in that** said device (1) further comprises a substrate (2) made of a material selected from the group consisting of: silicon, polysilicon, silicon dioxide, glass, SU8, AIN, Aluminum oxide and metals so that the nanostructure (3) of said device is grown on said substrate (2).

7. Self-generating voltage device (1), according to any of claims 1 to 6, **characterized in that** the nanostructure (3) is a nanosheet (3), preferably wherein the nanosheet shape is a polygon.

8. Self-generating voltage device (1), according to claim 7, wherein the nanosheet shape is a hexagon or a trapeze.

9. Method for generating a voltage for growing and/or differentiating cells via electrical stimulation comprising the following steps:
a) contacting at least an isolated cell with the surface (31) of the nanostructure (3) of the device (1) according to any of claims 1 to 8,
b) adding a culture medium to the at least an isolated cell of step a) which allows that the cell engage the nanostructure (3), preferably nanosheet, of the device (1); and
c) incubating the cell of step b)
wherein the differentiation and/or growing of the cells induce an electrical stimulus through the mechanical stress produced in the device (1) and wherein the method it is **characterized in that** there is not any external stimuli.

10. Method according to claim 9 wherein the cell is an electroconductive cell, preferably the electroconductive cell is selected from any of the list consisting of: muscle cells, myoblasts, neural cells, myocardial cells, osteoblasts, osteoclasts, stem cells and induced pluripotent stem cells.

11. Implant comprising the device (1) according to any of claims 1 to 8 for growing and/or differentiating cells on the surface (31) of said device.

12. Implant according to claim 11 wherein the implant is biocompatible by comprising a coating comprising in turn pharmaceutically acceptable polymers and/or functionalized with specific ligands having affinity for a target cell and/or maker molecules that allow tracking thereof.

## Patentansprüche

1. Vorrichtung (1) für selbsterzeugende Spannung zur elektrischen Stimulation von Zellen (11), vorzugsweise zu Zelldifferenzierung, -mobilität und/oder -wachstum, wobei die Vorrichtung mindestens eine Nanostruktur (3) aus einem piezoelektrischen Material umfasst, wobei die Nanostruktur (3 ) ein Seitenverhältnis von mehr als 100 aufweist und im Wesentlichen flach ist, sodass die Zelle (11) auf der Oberfläche (31) der Nanostruktur (3) verankert werden kann, sodass eine mechanische Belastung in der Nanostruktur (3) erzeugt wird, die aufgrund des piezoelektrischen Charakters der Nanostruktur (3) eine Spannung erzeugt, wobei die Vorrichtung **dadurch gekennzeichnet ist, dass** die Breite der Nanostruktur (3) entlang der Nanostruktur (3) abnimmt.

2. Vorrichtung (1) für selbsterzeugende Spannung nach Anspruch 1, **dadurch gekennzeichnet, dass** die Nanostruktur (3) hergestellt ist aus: ZNO oder ZNO+AIN oder in Polymer-eingebettetem ZnO und AIN.

3. Vorrichtung (1) für selbsterzeugende Spannung nach einem der Ansprüche 1 bis 2, **dadurch gekennzeichnet, dass** die Nanostruktur (3) eine Dicke zwischen 20 ±1,34 nm und 40 ±4,5 nm aufweist.

4. Vorrichtung (1) für selbsterzeugende Spannung nach einem der Ansprüche 1 bis 3, wobei die Nanostruktur (3) eine maximale Dicke von 100 nm aufweist.

5. Vorrichtung (1) für selbsterzeugende Spannung nach einem der Ansprüche 1 bis 4, **dadurch gekennzeichnet, dass** die Nanostruktur (3) eine konstante Dicke aufweist.

6. Vorrichtung (1) für selbsterzeugende Spannung nach einem der Ansprüche 1 bis 5, **dadurch gekennzeichnet, dass** die Vorrichtung (1) ferner ein Substrat (2) umfasst, das aus einem Material hergestellt ist, das aus der Gruppe ausgewählt ist, bestehend aus: Silizium, Polysilizium, Siliziumdioxid, Glas, SU8, AIN, Aluminiumoxid und Metallen, sodass die Nanostruktur (3) der Vorrichtung auf dem Substrat (2) gezüchtet wird.

7. Vorrichtung (1) für selbsterzeugende Spannung nach einem der Ansprüche 1 bis 6, **dadurch gekennzeichnet, dass** die Nanostruktur (3) eine Nanofolie (3) ist, wobei die Nanofolienform vorzugsweise ein Polygon ist.

8. Vorrichtung (1) für selbsterzeugende Spannung nach Anspruch 7, wobei die Nanofolienform ein Sechseck oder ein Trapez ist.

9. Verfahren zum Erzeugen einer Spannung zum Züchten und/oder Differenzieren von Zellen durch elektrische Stimulation, umfassend die folgenden Schritte:
a) Inkontaktbringen mindestens einer isolierten Zelle mit der Oberfläche (31) der Nanostruktur (3) der Vorrichtung (1) nach einem der Ansprüche 1 bis 8,
b) Hinzufügen eines Kulturmediums zu der mindestens einen isolierten Zelle aus Schritt a), das ermöglicht, dass die Zelle mit der Nanostruktur (3), vorzugsweise Nanofolie, der Vorrichtung (1) in Eingriff gelangt; und
c) Inkubieren der Zelle aus Schritt b)
wobei die Differenzierung und/oder das Wachstum der Zellen durch die in der Vorrichtung (1) erzeugte mechanische Spannung einen elektrischen Stimulus hervorrufen und wobei das Verfahren **dadurch gekennzeichnet ist, dass** keine externen Stimuli vorhanden sind.

10. Verfahren nach Anspruch 9, wobei die Zelle eine elektrisch leitfähige Zelle ist, wobei die elektrisch leitfähige Zelle vorzugsweise ausgewählt ist aus einer Liste, bestehend aus: Muskelzellen, Myoblasten, neuralen Zellen, Myokardzellen, Osteoblasten, Osteoklasten, Stammzellen und induzierten pluripotenten Stammzellen.

11. Implantat, umfassend die Vorrichtung (1) nach einem der Ansprüche 1 bis 8 zum Züchten und/oder Differenzieren von Zellen auf der Oberfläche (31) der Vorrichtung.

12. Implantat nach Anspruch 11, wobei das Implantat biokompatibel ist, indem es eine Beschichtung umfasst, die wiederum pharmazeutisch verträgliche Polymere umfasst und/oder mit spezifischen Liganden funktionalisiert ist, die eine Affinität für eine Zielzelle und/oder Markermoleküle aufweisen, die deren Verfolgung ermöglichen.

## Revendications

1. Dispositif autogénérateur de tension (1) pour la stimulation électrique de cellules (11), de préférence pour la différenciation, la mobilité et/ou la croissance cellulaire, le dispositif comprenant au moins une nanostructure (3) composée d'un matériau piézoélectrique, dans lequel la nanostructure (3) a un rapport d'aspect supérieur à 100 et est essentiellement plate de sorte que la cellule (11) peut s'ancrer sur la surface (31) de la nanostructure (3) de sorte qu'une contrainte mécanique est produite dans la nanostructure (3 ) générant une tension due au caractère piézoélectrique de la nanostructure (3), le dispositif étant **caractérisé en ce que** la largeur de la nanostructure (3) diminue le long de la nanostructure (3).

2. Dispositif autogénérateur de tension (1), selon la revendication 1 **caractérisé par** la nanostructure (3) étant composée de : ZNO, ou ZNO+AIN ou ZnO et AIN enrobés de polymère.

3. Dispositif autogénérateur de tension (1), selon l'une quelconque des revendications 1 à 2 **caractérisé en ce que** la nanostructure (3) a une épaisseur comprise entre 20 ± 1,34 nm et 40 ± 4,5 nm.

4. Dispositif autogénérateur de tension (1), selon l'une quelconque des revendications 1 à 3 dans lequel la nanostructure (3) a une épaisseur maximale de 100 nm.

5. Dispositif autogénérateur de tension (1), selon l'une quelconque des revendications 1 à 4 **caractérisé en ce que** la nanostructure (3) a une épaisseur constante.

6. Dispositif autogénérateur de tension (1), selon l'une quelconque des revendications 1 à 5 **caractérisé en ce que** ledit dispositif (1) comprend en outre un substrat (2) composé d'un matériau choisi dans le groupe constitué de : silicium, polysilicium, dioxyde de silicium, verre, SU8, AIN, oxyde d'aluminium et métaux de sorte que la nanostructure (3) dudit dispositif croît sur ledit substrat (2).

7. Dispositif autogénérateur de tension (1), selon l'une quelconque des revendications 1 à 6, **caractérisé en ce que** la nanostructure (3) est une nanofeuille (3), de préférence dans lequel la forme de la nanofeuille est un polygone.

8. Dispositif autogénérateur de tension (1), selon la revendication 7, dans lequel la forme de la nanofeuille est un hexagone ou un trapèze.

9. Procédé de génération d'une tension pour faire croître et/ou différencier des cellules par stimulation électrique comprenant les étapes suivantes :
a) mise en contact d'au moins une cellule isolée avec la surface (31) de la nanostructure (3) du dispositif (1) selon l'une quelconque des revendications 1 à 8,
b) ajout d'un milieu de culture à l'au moins une cellule isolée de l'étape a) qui permet que la cellule vienne en prise avec la nanostructure (3), de préférence la nanofeuille, du dispositif (1) ; et
c) incubation de la cellule de l'étape b)
dans lequel la différenciation et/ou la croissance des cellules induisent un stimulus électrique par le biais de la contrainte mécanique produite dans le dispositif (1) et dans lequel le procédé est **caractérisé en ce qu'**il n'y a aucun stimulus externes.

10. Procédé selon la revendication 9 dans lequel la cellule est une cellule électroconductrice, de préférence la cellule électroconductrice est choisie parmi l'une quelconque de la liste constituée de : cellules musculaires, myoblastes, cellules nerveuses, cellules du myocarde, ostéoblastes, ostéoclastes, cellules souches et cellules souches pluripotentes induites.

11. Implant comprenant le dispositif (1) selon l'une quelconque des revendications 1 à 8 pour la croissance et/ou la différenciation de cellules sur la surface (31) dudit dispositif.

12. Implant selon la revendication 11 dans lequel l'implant est biocompatible en comprenant un revêtement comprenant à la fois des polymères pharmaceutiquement acceptables et/ou fonctionnalisés avec des ligands spécifiques ayant une affinité pour une cellule cible et/ou des molécules productrices qui permettent son suivi.
